# EUROPEAN PATENT APPLICATION

(11) **EP 4 317 138 A1**
(43) Date of publication of application: **07.02.2024**
(21) Application number: 22928065.6
(22) Date of filing: 02.06.2022
(51) Int. Cl.: C07D 239/48, C12P 13/00

(54) **2,4-DIAMINOPYRIMIDINE OXIDE PREPARATION METHOD**

(30) Priority: 22.02.2022 CN 202210159583
(71) Applicant: Shanghai Bluepha Biosciences Co., Ltd., Shanghai 200444 (CN)
(72) Inventor: TAN, Chang, Shanghai 200444 (CN); LI, Teng, Beijing 102206 (CN); ZHANG, Haoqian, Shenzhen, Guangdong 518057 (CN); JIAN, Yimin, Shanghai 200444 (CN); TENG, Xin, Shanghai 200444 (CN)
(74) Representative: Kador & Partner Part mbB
(86) International application number: PCT/CN2022/096786
(87) International publication number: WO 2023/159794

(57) **Abstract**

The invention relates to the technical field of synthesis of a heterocyclic compound, and particularly discloses a method for preparing 2,4-diaminopyrimidine oxide. The method includes the steps of: cyclizing 3-oxopropionitrile with guanidine under an oxidizing condition to obtain 2,4-diaminopyrimidine oxide, wherein the 3-oxopropionitrile is obtained by oxidizing 3-hydroxypropionitrile, which is a cheap and easily available pharmaceutical chemical, as a raw material. The method of the present invention is green and mild, realizing the synthesis of Aminexil from a simple, cheap and easily available raw material.

## Description

### TECHNICAL FIELD

The invention relates to the technical field of synthesis of heterocyclic compounds, and in particular to a method for preparing 2,4-diaminopyrimidine oxide.

### BACKGROUND

2,4-Diaminopyrimidine oxide(trade name Aminexil), is an exclusive patented anti-hair loss ingredient developed by and widely used in shampoo and hair care products of L'Oreal Group. Many cases of hair loss are often accompanied with "perifollicular fibrosis", that is, the thickening and hardening of collagen fibers around the hair follicle, which impedes the insertion of the hair bulb into the deep dermis, causing compression of the follicle and blocking the delivery of nutrients, resulting in hair getting more and more fragile and soft. 2,4-Diaminopyrimidine, which is a relatively safe hair-fixing ingredient, can effectively soften the keratin around the hair follicle and prolong the life of the hair follicle without side effects, and its persistent use can achieve the purpose of long-term anti-hair loss.

US3644364, US3382247, CN87104693 etc. reported methods for preparing pyrimidine derivatives, but they all directly use a halogenated diaminopyrimidine as the substrate, and the products are all derivatives of Minoxidil. The synthetic route of Aminexil is not proposed.

US2416617, WO2008/98058, WO2007/84786, WO2012/109423 reported the preparation of 2,4-diaminopyrimidine (an Aminexil precursor), wherein the synthesis is mainly performed by ammonia substitution by using a halogenated nitrogen heterocyclic precursor. However, the raw materials are complex and expensive, and the synthesis reaction conditions are harsh.

An early literature (Journal of the American Chemical Society, 1950, 72, 2587-2593) reported that 2,4-diaminopyrimidine was synthesized by condensation of 3,3-diethoxypropionitrile with guanidine hydrochloride. However, the preparation of 3,3-diethoxypropanenitrile requires using acetonitrile, sodium methoxide, and ethyl formate as raw materials, and reacting with carbon monoxide under a high pressure of 40-50 atm (US4525310), and this method has harsh reaction conditions, high danger and high equipment requirements.

In view of the above, the present invention is proposed.

### SUMMARY OF THE INVENTION

An object of the present invention is to provide a method for preparing 2,4-diaminopyrimidine oxide.

In order to achieve the purpose of the present invention, the following technical solutions are adopted:
The invention provides a method for preparing 2,4-diaminopyrimidine oxide, including the steps of:
(1) oxidizing 3-hydroxypropionitrile to obtain 3-oxopropionitrile;
(2) cyclizing 3-oxopropionitrile with guanidine under an oxidizing condition to obtain 2,4-diaminopyrimidine oxide.

The present invention designs a novel synthesis route of Aminexil to produce Aminexil from 3-oxopropionitrile and guanidine (such as guanidine hydrochloride) under an oxidizing condition. The 3-oxopropionitrile may be obtained by oxidizing 3-hydroxypropionitrile, a cheap and easily available pharmaceutical chemical, as a raw material.

The synthetic route is shown below:

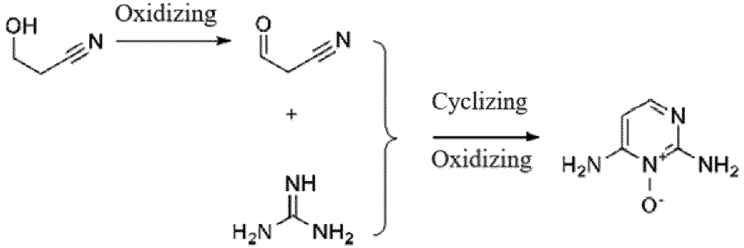

### Step (1)

The oxidizing may be performed using a chemical oxidant or a bio-oxidase as an oxidizing agent, that is, the oxidizing may be performed in the presence of a chemical oxidant or in the presence of a bio-oxidase without any particular limitation. Suitable conditions may be selected according to different oxidizing agents to prepare 3-oxopropionitrile, which may be also referred to as propionitrile aldehyde.

In some embodiments, the step (1) includes oxidizing 3-hydroxypropionitrile in the presence of a chemical oxidant to obtain 3-oxopropionitrile.

The chemical oxidant may be any chemical agent known in the art for oxidizing an alcohol to an aldehyde, including, but not limited to, hydrogen peroxide, KMnO₄, KClO₃, concentrated sulfuric acid, HNO₃, MnO₂, FeCl₃, etc., preferably hydrogen peroxide.

The molar ratio of the chemical oxidant to 3-hydroxypropionitrile may be 5:1 to 15:1, e.g., 5:1, 6:1, 8:1, 10:1, 12:1, or 15:1.

The oxidizing in the presence of a chemical oxidant may be performed under heating. The heating temperature may be 40-60 °C, e.g., 40, 50, or 60 °C, and the reaction time may be 2-6 h, e.g., 2, 3, 4, 5, or 6 h.

In some embodiments, the step (1) includes oxidizing 3-hydroxypropionitrile in the presence of a bio-oxidase to obtain 3-oxopropionitrile.

The bio-oxidase may be an aldehyde-tolerant broad-spectrum short-chain alcohol (generally refers to a C1-C6 alcohol) dehydrogenase or oxidase known in the art, such as ethanol dehydrogenase and ethanol oxidase.

The oxidizing in the presence of a bio-oxidase may be performed in a solution. The pH of the solution may be controlled at 7-8, preferably about 7.5. This can be achieved, for example, by using a phosphate buffer at a pH of about 7.5.

In some embodiments, the step (1) includes a step of purifying the obtained 3-oxopropionitrile. The purity of the final product can be improved by purifying the 3-oxopropionitrile for further reactions.

The purification may include the steps of: dissolving 3-oxopropionitrile in n-butanol, centrifuging, drying the supernatant with anhydrous sodium sulfate, adding concentrated sulfuric acid (for adjusting pH) and optionally, anhydrous copper sulfate (for promoting cyclization), sealing, heating overnight, centrifuging to leave the supernatant, extracting with sodium bicarbonate aqueous solution, and drying the upper organic phase with anhydrous sodium sulfate.

### Step (2)

In this step, 3-oxopropionitrile and guanidine undergo a cyclization reaction under an oxidizing condition to obtain 2,4-diaminopyrimidine oxide.

In order to reflect the reaction process more microscopically, this step can be split into two steps, and the changes of N before and after the reaction are as follows.

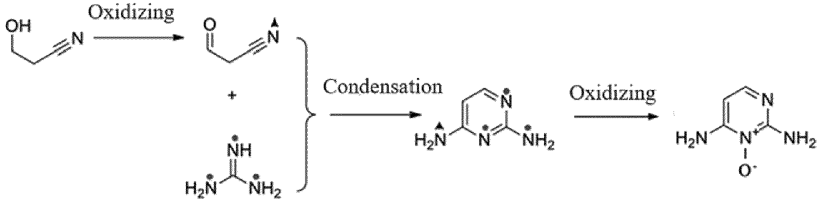

The guanidine may be performed using hydrochloride of guanidine (guanidine hydrochloride), nitrate of guanidine (guanidine nitrate), etc., preferably guanidine hydrochloride.

At this time, a base can be added to the reaction system to convert guanidine hydrochloride into guanidine and promote the cyclization reaction.

The base may be any agent known in the art for use as a base catalyst, including, but not limited to, lithium carbonate, lithium hydroxide, lithium t-butoxide, sodium carbonate, sodium bicarbonate, sodium hydroxide, sodium phosphate, sodium methoxide, sodium ethoxide, sodium isopropoxide, sodium t-butoxide, potassium carbonate, potassium bicarbonate, potassium hydroxide, potassium phosphate, potassium methoxide, potassium ethoxide, potassium t-butoxide, cesium carbonate, cesium hydroxide, magnesium carbonate, magnesium hydroxide, magnesium phosphate, magnesium oxide, magnesium methoxide, magnesium ethoxide, magnesium isopropoxide, magnesium t-butoxide, triethylamine, diisopropylamine, diisopropylethylamine, tri-n-butylamine, pyridine, 2-methylpyridine, 2,6-dimethylpyridine, 4-dimethylaminopyridine, tetrahydropyrrole, morpholine, piperidine, or 2,2,6,6-tetramethylpiperidine, or a combination thereof, preferably sodium carbonate, sodium hydroxide, potassium carbonate, potassium bicarbonate or potassium hydroxide, etc.

The cyclization reaction may be performed in a solvent.

The solvent may be a solvent known in the art that can act as a reaction medium for 3-oxopropionitrile and guanidine and does not react with 3-oxopropionitrile and guanidine, including, but not limited to, methanol, ethanol, isopropanol, n-butanol, isobutanol, isoamyl alcohol, dichloromethane, chloroform, benzene, toluene, xylene, chlorobenzene, acetonitrile, tetrahydrofuran, 2-methyltetrahydrofuran, dioxane, ethylene glycol dimethyl ether, methyl tert-butyl ether, isopropyl acetate, n-butyl acetate, phenyl methyl ether, ethanol, isopropanol, etc.

The oxidizing condition may be spontaneous oxidization in air, and a small amount of the oxidant remains in the process of preparing 3-oxopropionitrile will accelerate the oxidizing, and the oxidizing occurs together with the condensation.

In some embodiments, the molar ratio of guanidine to 3-oxopropionitrile may be 1:1 to 5:1, e.g.,1:1, 2:1, 3:1, 4:1, or 5:1, preferably 3:1.

If purification is performed in the step (1), the 3-oxopropionitrile here refers to the purified 3-oxopropionitrile.

By controlling the molar ratio of guanidine to 3-oxopropionitrile, the conversion of the reactants can be maximized. If the ratio is less than or greater than this ratio range, there will be a large amount of raw materials remaining, resulting in a decreased economic efficiency.

In some embodiments, the reaction temperature in the step (2) is 70-85 °C, preferably 77 °C; and the reaction time is 20-30 min. After mixing the two substrates (guanidine hydrochloride and 3-oxopropionitrile), the product may be seen as soon as 20 minutes, and the reaction is almost complete by 30 minutes.

The reaction temperature has an impact on the reaction rate. If it is less than or greater than the reaction temperature range, the reaction time will be prolonged, or the product content will be reduced.

In some embodiments, the method for preparing 2,4-diaminopyrimidine oxide includes the steps of:
Putting 3-hydroxypropionitrile into a centrifuge tube, adding a chemical oxidant, wherein the molar ratio of the chemical oxidant to 3-hydroxypropionitrile is 5:1 to 15:1, heating at 50 °C for 4 h, packaging and freeze-drying for 6 h, sealing and storing at -20 °C for further use to obtain freeze-dried 3-oxopropionitrile;
Dissolving the freeze-dried 3-oxopropionitrile in n-butanol, centrifuging, drying the supernatant with anhydrous sodium sulfate, adding concentrated sulfuric acid, sealing, heating overnight, centrifuging to leave the supernatant, extracting with an equal volume of an aqueous sodium bicarbonate solution, and drying the upper organic phase with anhydrous sodium sulfate to obtain a purified 3-oxopropionitrile; then adding a solution of guanidine hydrochloride and sodium ethoxide in ethanol, wherein the molar ratio of the guanidine hydrochloride to the purified 3-oxopropionitrile is 1:1 to 5:1, sealing, and heating at 77°C for 30 min.

In still some embodiments, the method for preparing 2,4-diaminopyrimidine oxide includes the steps of:
Mixing a substrate solution containing 3-hydroxypropionitrile with a bio-oxidase, reacting in a shaker, and drying to obtain 3-oxopropionitrile;
Dissolving the freeze-dried 3-oxopropionitrile in n-butanol, centrifuging, drying the supernatant with anhydrous sodium sulfate, adding anhydrous copper sulfate and concentrated sulfuric acid, sealing, heating overnight, centrifuging to leave the supernatant, drying the supernatant with anhydrous sodium sulfate to obtain a purified 3-oxopropionitrile; then adding a solution of guanidine hydrochloride and sodium ethoxide in ethanol, wherein the molar ratio of the guanidine hydrochloride to the purified 3-oxopropionitrile is 1:1 to 5:1, sealing, and heating at 77 °C for 30 min.

### Beneficial effect

The method of the present invention is green and mild, and realizes the synthesis of Aminexil from simple, cheap and easily available raw materials.

The present invention has been described above in detail, but the above embodiments are merely illustrative in nature and are not intended to limit the present invention. Furthermore, it is not limited herein by any theory described in the preceding prior art or the summary or the following examples.

Unless expressly stated otherwise, numerical ranges throughout the application include any sub-ranges therein and any numerical values incremented by the smallest subunit of the value given therein. Unless expressly stated otherwise, numerical values throughout the application represent approximate measures or limitations of ranges including minor deviations from the given values, as well as embodiments having about the recited value as well as having the exact recited value. Except in the working examples last provided in the detailed description, all numerical values of a parameter (e. g., amount or condition) in the present application documents (including the claims) are to be understood as being modified in all instances by the term "about", whether or not "about" actually appears before the numerical value. "About" means that the numerical value allows for some imprecision (some approximation in the value; approximately or reasonably close to the value; approximately). If "about" provides inaccuracy that is not understood in the art in its ordinary meaning, "about" as used herein means at least variations that can be produced by ordinary methods of measuring and using these parameters. For example, "about" may include a variation of less than or equal to 10%, less than or equal to 5%, less than or equal to 4%, less than or equal to 3%, less than or equal to 2%, less than or equal to 1%, or less than or equal to 0.5%.

### Brief Description of the Drawings

FIG. 1 shows a chromatogram of 3-oxopropionitrile obtained in example 1.
FIG. 2 shows a mass spectrum of 3-oxopropionitrile obtained in example 1.
FIG. 3 shows a chromatogram of diaminopyrimidine oxide obtained in example 3.
FIG. 4 shows a mass spectrum of diaminopyrimidine oxide obtained in example 3.
FIG. 5 shows a chromatogram of diaminopyrimidine oxide obtained in example 4.
FIG. 6 shows a mass spectrum of diaminopyrimidine oxide obtained in example 4.

### DETAILED EMBODIMENTS

The present invention will be further described in conjunction with the examples below. It should be noted that the following examples are provided for illustrative purposes only, and does not constitute a limitation on the protection scope of the present invention.

Unless otherwise specified, the raw materials, reagents, and methods used in the examples are all conventional raw materials, reagents, and methods in the art.

The chromatograph device used was an Agilent 1200.

The mass spectrometer used was a QTRAP 4500 triple quadrupole mass spectrometer (SCIEX).

### Example 1 Synthesis of 3-oxopropionitrile

200 µL of 3-hydroxypropionitrile was put into a 50 mL centrifuge tube, and added with 1.6 mL of 30% hydrogen peroxide, followed by addition of 60 µL of an aqueous ferric chloride solution (800 mM). The mixture was heated at 50 °C for 4 h, and then packaged into 3 tubes, freeze-dried for 6 h, sealed and stored at -20 °C for later use.

### Sample testing:

The sample was dissolved in 300 µL of n-butanol, and centrifuged. 2 µL of supernatant was taken and diluted 100 times with water. 10 µL of the diluent was taken, added with 10 µL of a DNPH derivatization reagent, and derivatized at 60 °C for 30 minutes, and then 20 µL of acetonitrile was added, and liquid chromatography and mass spectrometry detection were performed.

Preparation of the DNPH derivatization reagent: 4 mg of DNPH (2,4-dinitrophenylhydrazine) was dissolved in 4 mL of a solution (1 mL of concentrated hydrochloric acid, 0.5 mL of acetonitrile, and 2.5 mL of water) under ultrasonication, and stored at -20°C for later use.

### Chromatographic conditions:

Column: C18 reversed phase column;
Mobile phase: A (0.1% aqueous formic acid solution) : B (acetonitrile) = 40:60;
Flow rate: 1mL/min;
Detection wavelength: 360nm;
Retention time: 8.0min.

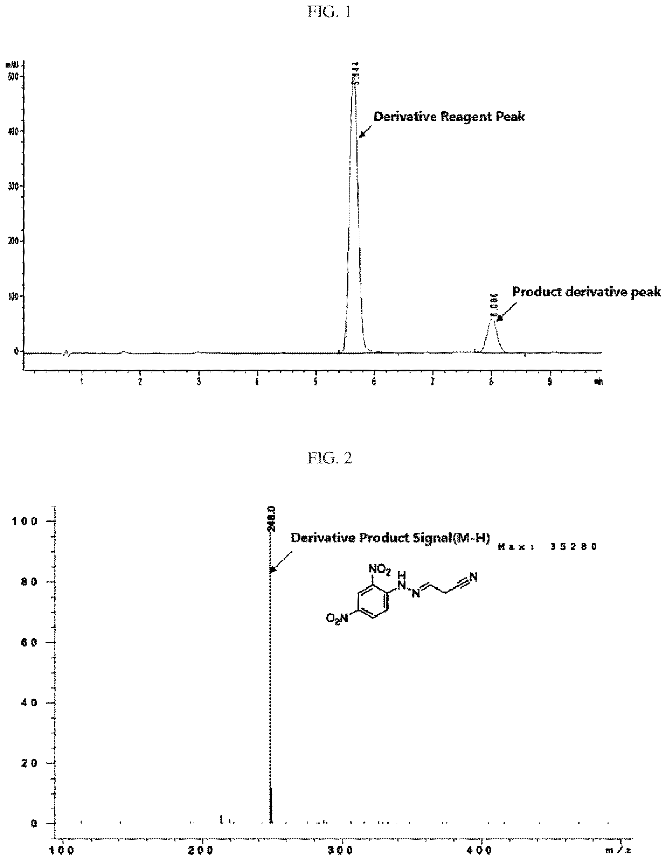

Detection results: The chromatogram is shown in Fig. 1. The mass spectrum is shown in Fig. 2. It was proved that the product contained 3-oxopropionitrile.

### Example 2 Synthesis of 3-oxopropionitrile

### 1. Preparation of bio-oxidase:

Glycerol tube bacteria liquid of *pichia pastoris* strain was streaked on a YPD solid medium for activation, and cultured for 3 days at 37 °C. A single clone was picked from the YPD plate, inoculated into 10 mL of a YPD liquid medium, cultured for about 24 hours at 30 °C, and transferred to 250 mL of a YPM liquid medium at 2%-5% inoculum. When OD=1, methanol (1% of the volume of the culture medium) was used to perform induction for 48 hours. After the bacteria grow to OD₆₀₀ₙₘ ≈ 8-9, the bacterial liquid was divided into 50mL centrifuge tubes, and centrifuged at 5000 rpm for 10 min. The supernatant was discarded, and the bacterial precipitation was stored at -20°C for use.

### Preparation of the culture media:

Preparation of the YPD culture medium (1 L): 10 g of yeast powder, 20 g of peptone, and 20 g of glucose. They were dissolved to a volume of 1 L, and sterilized at 115 °C for 30 min. For the solid culture medium, 1.5% of agar was further added.

Preparation of the YPM culture medium (1 L): 10 g of yeast powder, 20 g of peptone, and 50 g of maltose. Sterilization was performed at 115 °C for 30 min.

The bacteria were resuspended in 2-3 mL of a 0.25M phosphate buffer with pH 7.5 at a bacterial concentration of OD₆₀₀ₙₘ≈75. The cells were disrupted in an ultrasonic disruptor at a power of 30% for a disruption time of 10 min, and then centrifuged at 8000 rpm/min for 10 min. The cell debris were discarded, and the supernatant was divided into 2mL centrifuge tubes, added respectively with 1.5 volumes of acetone, refrigerated for 30min, and centrifuged at 12,000 rpm/min for 2 min. The supernatant was discarded, and the protein precipitation was added with the 0.25 M phosphate buffer solution having a volume equivalent to 1/2 volumes of the initial cell disruption supernatant for reconstitution, and centrifuged at 12,000 rpm/min for 2 min. The insoluble matter was discarded, and the supernatant (oxidase solution), which contains the oxidase using an alcohol as the substrate, was packaged and stored at -20°C for later use.

### 2. Preparation of 3-oxopropionitrile:

3-Hydroxypropionitrile was used as the substrate, and the oxidase-containing supernatant prepared in the above step 1 was used to oxidize the hydroxyl of the substrate to aldehyde.

10 mL of a substrate solution was prepared according to the ratio given in Table 1. The substrate solution and the above oxidase solution were mixed in a volume ratio of 1:1, and then reacted on a shaker at 30 °C for 4 hours. The product was sampled to detect the content of 3-oxopropionitrile by using the same method as example 1. 2.1 mM of 3-oxopropionitrile was generated, freeze-dried for 6 h, sealed and stored at -20°C for later use.

**Table 1**

| Substrate solution | Concentration of stock solution | Volume | Final concentration |
|---|---|---|---|
| pH 7.5 phosphate buffer | 1 M | 5000 µL | 0.5 M |
| 3-hydroxypropionitrile | 14 M | 140 µL | 0.2 M |
| Catalase | 60000 U/mL | 10 µL | 60 U/mL |
| Water | - | 4850 µL | - |

### Example 3 Synthesis of diaminopyrimidine oxide

A tube of freeze-dried 3-oxopropionitrile prepared in Example 1 was dissolve in 300 µL of n-butanol, and centrifuged. The supernatant was dried with anhydrous sodium sulfate for three times, added with 9 µL of concentrated sulfuric acid for adjusting pH, sealed, heated at 77 °C overnight, and centrifuged. The supernatant was extracted three times with an equal volume of a 0.2 M aqueous sodium bicarbonate solution, and the upper organic phase was dried three times with anhydrous sodium sulfate to obtain a purified 3-oxopropionitrile, which was then added with 10 mg of guanidine hydrochloride (the molar ratio of the guanidine hydrochloride to the purified 3-oxopropionitrile was 3:1) and 50 µL of a 20% ethanol solution of sodium ethoxide, sealed and heated at 77 °C for 30 min, and the product was detected.

**Sample detection:** The sample was diluted 50 times with water and then directly detected by LC-MS.

### Chromatographic conditions:

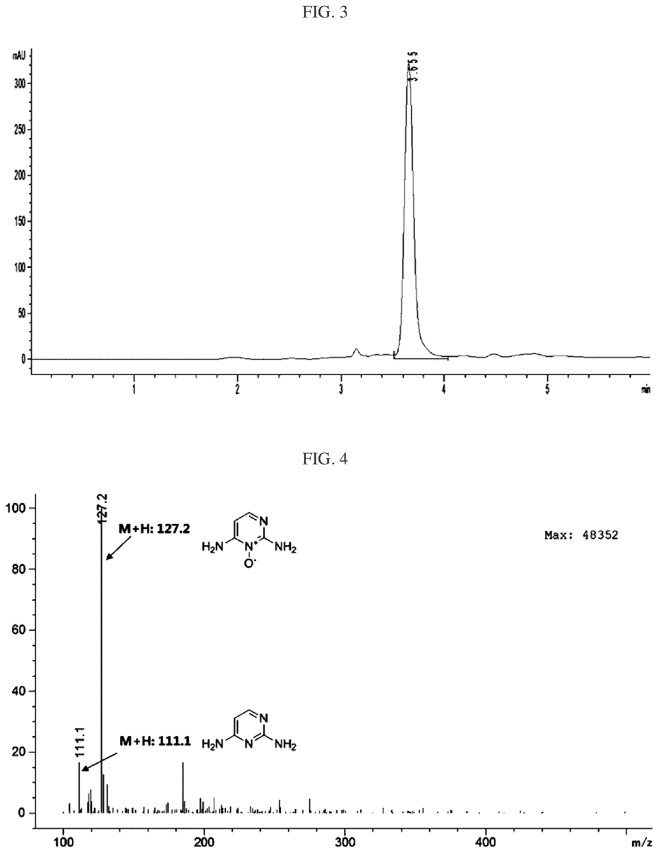

Column: C18 reversed phase column;
Mobile phase: A (0.1% aqueous formic acid solution) : B (acetonitrile) = 98:2;
Flow rate: 1 mL/min;
Detection wavelength: 254 nm;
Retention time: 3.7 min.
Detection results: As shown in Figs. 3 and 4, most of the product was the oxide, with a small part unoxidized.

### Example 4 Synthesis of diaminopyrimidine oxide

A tube of freeze-dried 3-oxopropionitrile prepared in Example 2 was dissolve in 300 µL of n-butanol, and centrifuged. The supernatant was dried with anhydrous sodium sulfate for three times, added with 30 mg of anhydrous copper sulfate for promoting cyclization and 9 µL of concentrated sulfuric acid for adjusting pH, sealed, heated at 77 °C overnight, and centrifuged. The supernatant was dried twice with anhydrous sodium sulfate, added with 10 mg of guanidine hydrochloride and 80 µL of a 20% ethanol solution of sodium ethoxide, sealed, and heated at 77 °C for 30 min, and the product was detected.

**Sample detection:** The sample was diluted 50 times with water and then directly detected by LC-MS.

Detection conditions: the same as Example 3.

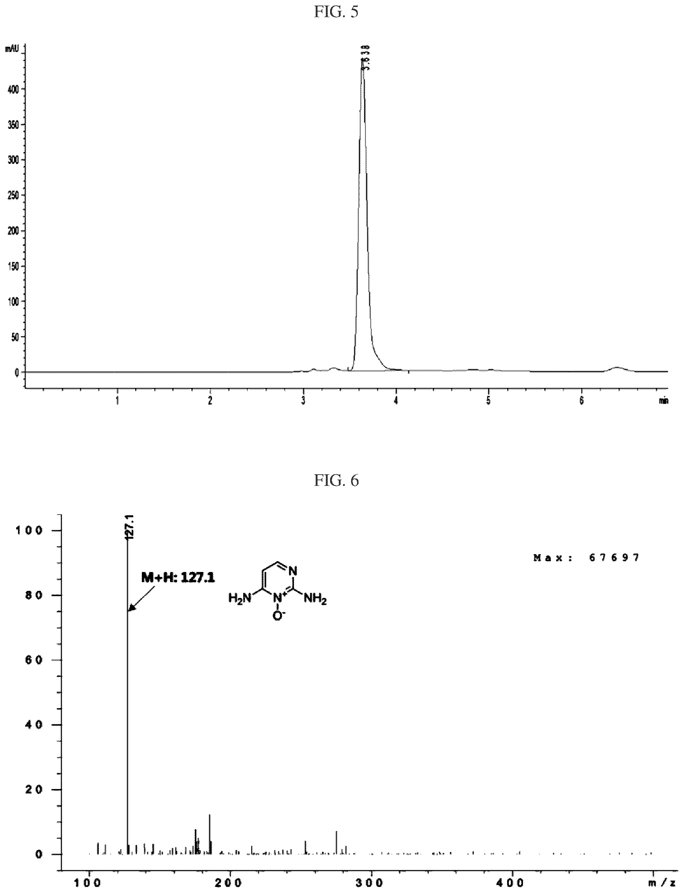

Detection results: As shown in Figs. 5 and 6, most of the product was the oxide, with a small part unoxidized.

The above examples are only to illustrate the technical solutions of the present invention, and the present invention is not limited thereto. Although the present invention has been described in detail with reference to the above examples, those skilled in the art should understand that the technical solutions described in the above examples can be modified, or some or all of the technical features therein can be equivalently replaced without departing from the spirit and essence as defined by the claims of the present invention, and these modifications or replacements are still within the scope defined by the claims of the present invention.

## Claims

1. A method for preparing 2,4-diaminopyrimidine oxide, including the steps of:
(1) oxidizing 3-hydroxypropionitrile to obtain 3-oxopropionitrile;
wherein the oxidizing is performed in the presence of a chemical oxidant under heating; the chemical oxidant is one or more selected from the group consisting of hydrogen peroxide, KMnO₄, KClO₃, concentrated sulfuric acid, HNO₃, MnO₂, FeCl₃, and the heating temperature is 40-60 °C; or
the oxidizing is performed in the presence of a bio-oxidase, the bio-oxidase is selected from aldehyde-tolerant broad-spectrum C1-C6 alcohol dehydrogenases and C1-C6 alcohol oxidases;
(2) cyclizing 3-oxopropionitrile with guanidine under an oxidizing condition to obtain 2,4-diaminopyrimidine oxide.

2. The method according to claim 1, wherein the molar ratio of the chemical oxidant to 3-hydroxypropionitrile is 5:1 to 15:1.

3. The method according to claim 1, wherein the reaction time of the oxidizing in the presence of a chemical oxidant is 2 to 6 h.

4. The method according to claim 1, wherein the molar ratio of the guanidine to 3-oxopropionitrile in step (2) is 1:1 to 5:1.

5. The method according to claim 4, wherein the reaction temperature in step (2) is 70 to 85 °C and the reaction time is 20 to 30 min.

6. The method according to claim 1, wherein the guanidine of step (2) is at least one selected from the group consisting of guanidine hydrochloride and guanidine nitrate.

7. The method according to claim 1, wherein the step (1) includes a step of purifying the 3-oxopropionitrile.
